# EUROPEAN PATENT SPECIFICATION

(11) **EP 0 895 473 B1**
(45) Date of publication and mention of the grant of the patent: **21.11.2001**
(21) Application number: 97908411.8
(22) Date of filing: 24.03.1997
(51) Int. Cl.: A61K 9/16, A61K 9/00, A61K 38/28, A61K 38/23, A61K 31/135, A61K 31/485, A61K 38/27, A61K 9/50

(54) **POLYSACCHARIDE MICROSPHERES FOR THE PULMONARY DELIVERY OF DRUGS**
MIKROKUGELN AUS POLYSACCHARID ZUR PULMONAREN WIRKSTOFFABGABE
MICROSPHERES DE POLYSACCHARIDES POUR L'ADMINISTRATION DE MEDICAMENTS DANS LES VOIES RESPIRATOIRES

(30) Priority: 23.03.1996 GB 9606188
(43) Date of publication of application: 10.02.1999
(73) Proprietor: West Pharmaceutical Services Drug Delivery & Clinical Research Centre Limited, Nottingham NG7 2TN (GB)
(72) Inventor: ILLUM, Lisbeth, The Park Nottingham NG7 1BA (GB); WATTS, Peter, James, West Bridgford Nottingham NG2 6DR (GB)
(74) Representative: Bassett, Richard Simon
(86) International application number: GB9700808
(87) International publication number: WO9735562

(56) References cited:
- EP-A- 0 611 567
- WO-A-93/15737
- WO-A-96/03978
- WO-A-96/09814

## Description

This invention relates to novel polysaccharide microsphere compositions. In particular it relates to compositions for use in the delivery of therapeutic agents which cannot readily be delivered orally, such as anti-asthma compounds, peptides, proteins, heparin and derivatives thereof, antisense agents and genes, to the lung of a mammal for local treatment and/or for systemic effect.

### Background and Prior Art

Drugs can be delivered to the lung for local effect, for example in treatment of asthma. Drugs which are known to act locally include bronchodilators, sodium cromoglycate and steroids. These substances are usually delivered to the central airways.

The lungs can also be used to deliver drugs into the general blood circulation for systemic effect. Well known examples include the anaesthetic gases and nicotine (from inhaled tobacco smoke).

Most conventionally, however, drugs are delivered systemically *via* the oral route. Provided a drug has an adequate lipid solubility it can be well transported into the blood from the gastrointestinal tract by a process of passive diffusion. Further, a small number of drugs can be absorbed by an active transport mechanism.

However, there are now an increasing number of drugs that cannot readily be given orally since they are highly polar in nature, are of a large size and/or have stability problems under the conditions prevalent in the gastrointestinal tract (e.g. acid pH in the stomach and/or endogenous enzymes in the small intestines). Examples of agents which cannot readily be delivered *via* this route include the products of biotechnology (in the form of therapeutic proteins (such as granulocyte colony stimulating factor, erythropoietin, interferons and growth hormone)) as well as polypeptide drugs produced by synthesis (such as calcitonins, parathyroid hormone, desmopressin, LHRH analogues (buserelin, goserelin and nafarelin, cholecystokinin and atrial naturetic peptide). Insulin can be considered to be the best known drug which exhibits this problem.

Other examples of compounds which demonstrate poor absorption from the intestines are polysaccharide materials such as heparin (and its low molecular weight derivatives), antisense agents, polar metabolites of opioid analgesics (morphine-6-glucuronide). Certain other drugs, when given orally, may be absorbed *via* the intestines, but are extensively metabolised in the wall of the intestines or the liver; this is termed "the first pass effect".

Thus, the skilled person is presented with the problem of the provision of an alternative or a more effective delivery means for use with *inter alia* the abovementioned "challenging" therapeutic agents.

In previous patent applications the applicants have described how molecules including some of those mentioned above may be delivered *via* the nasal and vaginal routes. The applicants have now found that it is possible to deliver such molecules to the lungs for local and systemic treatment by incorporation (i.e. encapsulation) of drug inside a polysaccharide microsphere *via* a novel process, described hereinafter.

It is known to those skilled in the art that drugs may be well absorbed *via* the lungs; even polar molecules such as peptides and proteins are quite well absorbed (40% or better appearing in the blood) - when delivered to the peripheral airways. The prior art has been reviewed by Niven *et al* Pharmaceutical Research. **13** 1242 (1995). In measuring the effectiveness of the absorption of a drug *via* the pulmonary route, studies are usually conducted *via* animal experiments, which typically install solutions of drugs directly into the lungs. This, of course, is impractical for therapy in man.

In the delivery of polar drugs to human patients, nebuliser systems are used, in which a mist of drug solution is inhaled into the lungs over an extended period (about 10 - 15 minutes), This is also an effective means of delivering a dose of drug to the lower (peripheral) airways. However, this method of dosing suffers from the disadvantage that it is unpopular with patients because of the time required to administer drug. Moreover, the process of nebulisation is also known to cause degradation of certain drugs.

There thus exists a need for an effective means for delivering such drugs in a pulsatile or controlled fashion to the lungs. Various devices exist for this purpose, such as metered dose inhalers (MDIs), which are usually based on a volatile propellant such as a CFC liquid (or a newer, non-CFC, alternative), and dry powder devices.

However, the problem with all such devices is that the quantity of drug reaching the lungs is quite small (20% or less in many cases). Moreover, the quantity reaching the peripheral airways (for good absorption into the blood) can be less than 10%, and is indeed more often less than 1%. Hence, even though a drug may be well absorbed from the lower (peripheral) regions of the lung, currently available devices are often not able to deliver a sufficient quantity to this area, in order to produce the desired therapeutic effect.
Various attempts are being made to overcome this deposition problem and new devices have been described in the scientific and patent literature. Powder devices are popular in this regard.

Notwithstanding the mechanical properties of the device, and the spacer system which may be used with such a device, the problem also exists that the powder system containing the drug needs to have appropriate properties to enable effective deposition.

It is well known to those skilled in the art that solid particles intended for delivery to the lungs should be of an aerodynamic diameter (as defined in *"Aerosols in Medicine",* Morén *et al*, Elsevier (1993), at page 64) of less than 10 microns and, preferably, of less than 5 microns. However, the particles should not be too small, or the particles will fail to deposit in the lung and be exhaled. A size range of 0.5 to 5 microns is thus preferred. Complex drugs such as peptides and proteins, low molecular weight heparin, antisense agents, DNA, may be micronised in order to produce this size range, but this process is known to cause damage to labile molecules. Moreover, physical losses can occur during the active processing operation.

In addition, many of the products of biotechnology, in the form of peptides and proteins, must be administered in very low doses (e.g. less than 1 mg). The pharmaceutical formulation can therefore suffer from problems of dose size and dose uniformity. Such drugs may be mixed *via* an appropriate means with an inert carrier, and lactose and mannitol are often used for this purpose (see, for example, WO 95/31479). However, problems associated with dose uniformity and segregation of the active material may still be exhibited. Furthermore, certain drugs are known to be surface active, or have properties than can greatly affect the electrostatic and flow properties of simple mixed systems.

In summary therefore, in view of the inadequate nature of currently available powder systems, and the poor performance of such systems in powder devices. a major problem exists in relation to the effective delivery of the abovementioned "challenging" drugs to the lung by way of powder systems. We have surprisingly found that microspheres prepared from soluble polysaccharides prepared by way of a spray drying process can overcome these problems.

Microspheres for administration to the lung have also been reviewed by Zeng *et al* (see International Journal of Pharmaceutics, **107,** 205 (1994) and **124,** 149 (1995)). Albumin microspheres for lung delivery have also been described in CA 2036844 and by Zeng *et al* (International Journal of Pharmaceutics, **109,** 135 (1994)). In these documents, microspheres are prepared by a conventional method involving emulsification and cross-linking. Solid microspheres, prepared by such a cross-linking process, are expected to have unsatisfactory degradation properties in the lung. Moreover, the cross-linking of the carrier in the manner described in this prior art in the presence of a sensitive drug, such as a peptide or protein, is also expected to cause chemical modification of that drug. The preparation of cross-linked microspheres of polysaccharides for controlled release by emulsifying vinyl derivatives of hydrophilic polymers is described in EP 245820 and by Artursson *et al* (J. Pharm. Sci. **73**, 1507 (1984)). Questions can be raised concerning biodegradation and safety for reasons including those discussed below.

Starch microspheres cross-linked with epichlorohydrin produced *via* an emulsification process have been reported for the nasal administration of drugs. Whilst the majority of the microspheres can be degraded (for example by amylases), a small amount of cross-linked material, when produced in this way always remains in the nasal cavity. Whilst this is of no concern in nasal delivery, it is expected to lead to adverse effects in the lung, especially if the particles were delivered to the alveolar regions where they would not be cleared by the mucociliary clearance process.

The administration of lactide/glycolide copolymer nanospheres for pulmonary delivery of peptide drugs such as LHRH analogues has been reported by Niwa *et al* (Yakugaku Zasshi, Journal of the Pharmaceutical Society of Japan. **115,** 732, (1995)). The particles were prepared by an emulsification process.

In a previous patent application (WO 93/02712) the applicants have described how hollow and solid microspheres can be prepared using a double emulsification process from soluble starch. It was suggested that such particles could be useful for the delivery of drugs to the lung. Microporous spherical particles have been described in US 4818542. The agents mentioned here included starch. A pore incorporating agent was also included in the formulation process.

Spray dried microparticles have been described in the prior art. Spray dried soluble proteins are described in EP 315875, WO 92/18164, WO 94/08627. In WO 94/08627, wall forming materials for hollow microcapsules included polysaccharides of low solubility. The use of such microspheres for delivery to the lung was not disclosed. Spray drying of pregelatinized starch and of pregelatinized hydroxypropyl starch is described in US 4871398 and US 4847371 respectively. In neither case is the use of spray drying of soluble polysaccharides for the preparation of drug loaded particles for pulmonary administration described. In EP 611567, sustained-release micropartides for pulmonary delivery are produced by spray drying a drug in the presence of hydroxypropyl cellulose and/or hydroxypropyl methyl cellulose; the formation of microspheres is not mentioned.

WO 96/03978 discloses the preparation by a process of spray drying of solid, glassy vehicles for drug administration principally from carbohydrates.

### Detailed Description of the Invention

We have found surprisingly that, by using certain soluble polysaccharides, such as starches, and a spray drying process, in which drug is mixed in aqueous solution with the polysaccharide material prior to spraying, it is possible to produce, in one step, microspheres in which drug is incorporated (i.e. encapsulated). that have the preferred size range for good lung deposition.

According to the invention there is provided a composition for the delivery of pharmacological agents to the respiratory tract of a mammal to provide peripheral deposition and systemic uptake wherein the therapeutic agent is incorporated into a polysaccharide through a process of spray drying a mixture of said agent and polysaccharide, which polysaccharide is either in aqueous solution or in the aqueous phase of an emulsion, and wherein the polysaccharide is selected from soluble starch, amylodextrin, hydroxyethyl starch, polyglucosamine, amylopectin, amylose, dextran, pullulan, carboxymethyl cellulose, carboxymethyl pullulan, diethylaminoethyldextran or a mucopolysaccharide, which compositions are hereinafter referred to as "the compositions according to the invention".

The compositions according to the invention are characterised by virtue of the fact that they are microspheres. The term "microspheres" will be well understood by those skilled in the art. The term thus includes those microparticles of a substantially spherical nature, and excludes those of a substantially granular and/or non-spherical nature, which latter particles may be made by mixing pharmaceutical agents with carriers or bulking agents by a suitable process, followed, if necessary, by further processing (e.g. pulverizing, micronising) to form a powder. In these contexts, by "substantially" we mean greater than 80%, preferably greater than 90%, spherical, granular and non-spherical, respectively.

The term polysaccharide will be well understood by those skilled in the art to exclude disaccharides, such as lactose. The compositions according to the invention comprise polysaccharides which have a molecular weight between 10,000 and 1,000,000, preferably between 50,000 and 75,000 and particularly between 100,000 and 300,000, and which are soluble in water. By "soluble in water" we mean that a solution of the polysaccharide can be prepared in aqueous solution at a concentration of 1 mg/ml or greater.

The compositions according to the invention have excellent flow properties and have the considerable adavantage that they exhibit good (i.e. high) and uniform loading of drugs. Although the compositions according to the invention permit loadings of drug as high as 50%, by "high loading of drugs" we mean a drug loading of greater than 10%. Furthermore, the compositions according to the invention have an improved performance *in vivo*, as determined in studies using human volunteers. When administered in a powder device, the compositions according to the invention exhibit superior properties when compared to a formulation prepared by the micronization of the drug to a size suitable for administration to the lung and its admixture with the carrier material lactose.

Microspheres may be formed in one step by spray drying a mixture of drug and soluble polysaccharide in solution.

According to a further aspect of the invention there is provided a method for preparing microspheres for the delivery of pharmacological agents to the respiratory tract of a mammal wherein the said agent is incorporated into a microsphere using a one step process where the drug is mixed in solution with a soluble polysaccharide and thereafter particles formed through a process of spray drying, characterised in that the polysaccharide is selected from soluble starch, amylodextrin, hydroxyethyl starch, polyglucosamine, amylopectin, amylose, dextran, pullulan, carboxymethyl cellulose, carboxymethyl pullulan, diethylaminoethyldextran or a mucopolysaccharide, which method is hereinafter referred to as "the process according to the invention".

The microspheres prepared by the process according to the invention also possess considerable advantages when compared to microspheres formed from polysaccharides as described in the prior art, by virtue of the fact that they may be prepared by a one step process. The microspheres prepared by the process according to the invention also have the advantage that they can be collected as a product that can be used without further processing. Moreover, the particle size distribution is narrow.

The compositions according to the invention may be prepared by spray drying aqueous solutions of polysaccharide or polysaccharide in an emulsion system.

When an emulsion system is used, microspheres can be prepared by spray drying polysaccharide in single (w/o) or (o/w) or double (w/o/w) (o/w/o) emulsion systems, the oil phase consisting of a volatile water immiscible solvent such as chloroform, methylene chloride and/or perfluorocarbons. In the emulsion systems the drug can either be dissolved in the water phase (hydrophilic drugs) or the oil phase (lipophilic drug).

However, a preferred method of producing drug loaded microspheres is as follows:

The drug is dissolved in water to produce a concentrated solution. The amount of drug employed will depend on the dose of drug that will be required in the final dose of the microsphere formulation and may vary from 0.1 mg to 2 g, more typically 1 g, of drug dissolved in 10 ml of distilled water.

The soluble polysaccharide is also dissolved in distilled water. The quantity of polysaccharide used will depend on its gelation and rheological properties, and can be varied from 0.1 g to 20 g in 200 ml, though typically 1 gram of polysaccharide will be dissolved in 20 ml of distilled water. For charged polysaccharides the pH and ionic strength of the solution can be adjusted by an appropriate means (such as those described hereinafter), but with clear recognition of the fact that the resultant microspheres will be delivered into the lung and that excessive quantities of electrolyte could alter the swelling and drug release properties of the final product.

The drug and polysaccharide solutions may then be combined. Preferred concentrations of polysaccharide in the combined solution are between 0.5 and 5 g per 20 ml and especially preferred concentrations are in the range 0.75 - 3 g per 20 ml. The viscosity of the resultant drug/polysaccharide solution should be suitable for dispersion in the selected spray drying device. A solution viscosity in the range 1 - 15 centipoise is preferred. For a non-Newtonian system such viscosity is measured as an apparent viscosity at a shear rate of 100 sec⁻¹.

The drug/polysaccharide solution can then be dispersed into microspheres using a suitable spray drying apparatus. Suitable apparatuses include that described hereinafter in the examples (i.e. the LabPlant SD-05 equipment available from LabPlant, Huddersfield, UK). Other suitable equipment which may be employed include the apparatus available form Buchi in Switzerland. The operating conditions such as the flow rate of the solution into the spray dryer, the size of the nozzle, the inlet and outlet air temperature, the atomization pressure and the flow rate of the drying air can be adjusted in accordance with the appropriate manufacturer's guidelines in order to provide the required particle size and release properties for the resultant microspheres. Such optimization conditions can be easily selected by the person skilled in the art of pharmaceutical formulation paying proper attention to known methods of experimental design. However, when the LabPlant SD-05 is used, preferred conditions are as follows: an inlet air temperature of between 100 and 200°C; an outlet air temperature of between 50 and 100°C; and spray rate of between 1 and 20 ml/min; a drying air flow of between 8 and 28 m³/h; an atomizing pressure of between 1 and 4 bar; and a nozzle size of between 0.1 and 2 mm. By using such methods, particles with a size appropriate for deposition in the different regions of the lung (i.e. an aerodynamic diameter of less than 10 microns, see above) and a narrow size distribution can be achieved.

The abovementioned spray drying process may, of course, also be used to prepare microspheres from polysaccharides in an emulsion system: However, when the preferred non-emulsion process is used, the compositions according to the invention have the additional advantage that they are not contaminated with solvents or oils used, as is the case in methods based on emulsification.

The compositions according to the invention can be administered to the lung in quantities from 1 - 100 mg of microspheres using an appropriate powder device. A preferred quantity of microspheres is in the range 5 - 50 mg. The drug content of the microsphere may range from a loading of less than 1% w/w of the microsphere to more than 50% w/w loading. The level of loading will, of course, be dictated by the therapeutic activity of the drug, the quantity of microspheres that can be delivered to the lung by the selected device, and the effect of the drug on the physical properties of the microsphere. Typically, loading will be between 1 and 10% w/w of drug to microsphere.

According to a further aspect of the invention, there is thus provided a method for the improved systemic delivery of pharmacological agents to a mammal by the respiratory tract, which comprises administering a composition according to the invention to a patient.

The compositions according to the invention have been found to gel and dissolve in the lung and are entirely biocompatible. In particular, when compositions according to the invention are prepared, which do not include the crosslinking agent or starch gel modifiers mentioned below, they are characterised by virtue of the fact that they may be dissolved rapidly (e.g. in less than five minutes, usually less than two minutes) and completely (e.g. a solution is formed at a concentration of 1 mg/ml or greater), when placed in water.

The compositions according to the invention may be prepared using different soluble polysaccharides including amylodextrin, amylopectin, hydroxyethylstarch, carboxymethylcellulose, diethylaminoethyldextran, dextran, pullulan, carboxymethyl pullulan or polyglucosamine. We also include mucopolysaccharides such as hyaluronic acid in this definition. Preferred polysaccharides include hydroxyethylstarch.

We have also found that, by varying the concentration of the soluble polysaccharide, and using different processing conditions, such as degree of crosslinking or addition of starch gel modifiers, it is possible to provide particles with different sizes and different release characteristics. Thus, in this manner, the drug can be released rapidly (for example to provide early appearance in the blood) or slowly (for example if the drug is required for local effects in the lung tissue or lung microcirculation).

By "released rapidly" we mean immediate release following delivery to the lung, which includes release of more that 50%, preferably more than 70%, and more preferably more than 80%, of drug just after (i.e. up to 5 minutes after) delivery to the lung. By "released slowly" we mean controlled release over a period of up to 6 hours following delivery to the lung.

However, we prefer that drug is released rapidly to the lung following delivery.

It is thus feasible to mix the polysaccharide solution with other excipients such as to provide a controlled release of the drug. Examples of such excipients are phospholipids, cyclodextrins, gelatin, alginate. Cross-linking agents may also be used to provide a controlled release of drug but, when this is the case it/they is/are chosen from material(s) that will provide total biodegradation of the microparticles. Polyphosphates are particularly preferred for this purpose for use with polysaccharides. Alternatively, aldose sugars can be used for polyglucosamines. Appropriate starch gel modifyers for use in the compositions according to the invention include fatty acids, preferably sodium myristate and monoglycerides.

In the compositions according to the invention, more than 80%, and preferably more than 90%, of microspheres have an aerodynamic diameter, or a particle size, of between 0.1 to 10 *µ*m, more preferably between 0.5 to 5 *µ*m, as measured by a Malvern Mastersizer or by optical microscopy.

The novel microspheres can, depending upon the preparation method, be loaded with lipophilic drugs or more especially, water soluble drugs. By "water soluble drugs" we mean that a solution of the drug can be prepared in the solution of soluble polysaccharide at a concentration of 1 mg/ml or greater. Examples include insulin, calcitonin, parathyroid hormone, cholecystokinin, desmopressin, leutinizing hormone releasing hormone and analogues thereof, human growth hormone, growth hormone releasing hormone, interferon (alpha, beta, consensus), leptin, somatostatin, superoxide dismutase, erythropoietin, colony stimulating factors, oligonucleotides, heparin, or a low molecular weight derivative thereof (by "low molecular weight" we mean a molecular weight of less than 10,000), DNA, analgesics (including polar analgesics such as morphine and metabolites thereof (including polar metabolites such as the glucuronides of morphine). By "polar" we mean a molecule with partition coefficient (octanol-water system) of less than 100. Other drugs and the salts of drugs which can be used include drugs for asthma treatment, immunomodulators, nicotine salts, soluble salts of salbutamol, terbutaline, and sodium cromoglycate. Antihistamines such as azatadine maleate, diphenhydramine hydrochloride, cardiovascular drugs such as diltiazem hydrochloride, timolol maleate, analgesic agents such as pethidine hydrochloride, hydromorphine hydrochloride, propoxyphene hydrochloride and tranquilisers such as promazine hydrochloride may also be used. Other examples of active pharmacological ingredients of high solubility in water are listed in US 5202128 and are included herein by reference.

Proteins for local treatment. which can also be incorporated into the microspheres include monoclonal and polyclonal antibodies, alpha 1 - antitrypsin, deoxyribonuclease. The protein drugs described above can also be administered as their chemical conjugates with polyethylene glycol.

Combinations of any of the abovementioned drugs may also be used.

The microsphere powders produced in the present invention may be used in a suitable dry powder device familiar to those skilled in the art. These include, but are not limited to, the Spinhaler™ (Fisons plc), Lyphodose™ (Valois S.A.), Monopoudre™ (Valois S.A.), Valois DPI™ (Valois S.A.), Turbospin™ (Phildeatech), multichamber powder inhaler (Pfeiffer), Turbohaler™ (Astra-Draco AB), Rotahaler™ (Glaxo), Diskhaler™ (Glaxo), Pulvinal™ (Chiesi Farmaceutici SpA) and Ultrahaler™ (Fisons).

Additionally, the microspheres may be lightly compacted to produce a solid compact from which a dose is taken *via* a mechanical method (eg. Ultrahaler™, Fisons). If necessary the microspheres can also be mixed with a small amount of excipients such as lactose to improve flow properties.

The invention is illustrated, but in no way limited, by way of the following examples.

### Brief Description of the Figures

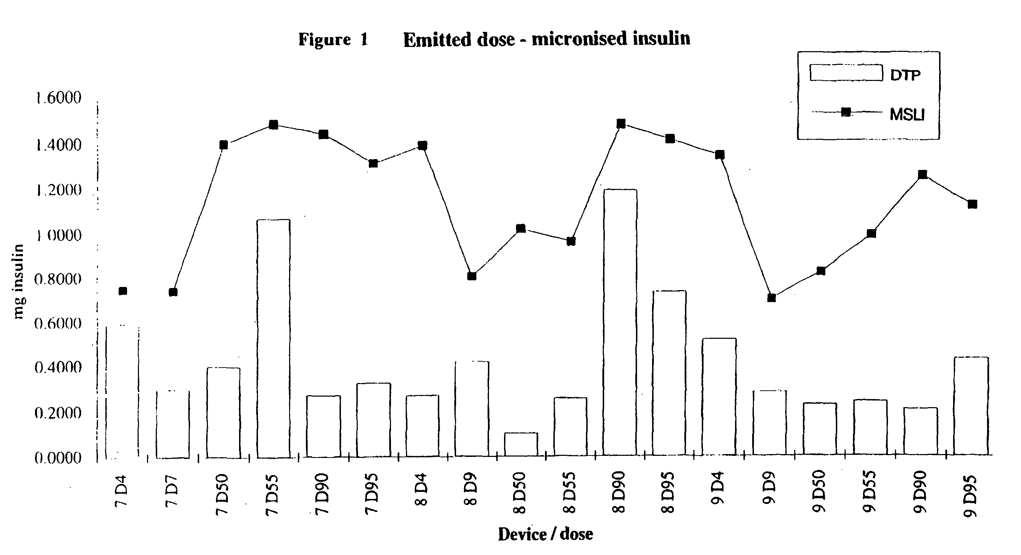

Figure 1 shows an emitted dose plot for micronised insulin, dispensed from a Fisons Ultrahaler™, as determined using an Emitted Dose Apparatus (Multi-stage Liquid Impinger (MSLI)) in which the amount of dose to the MSLI, and the dose to patient (DTP; calculated from the MSLI), are plotted against dose number/device number.

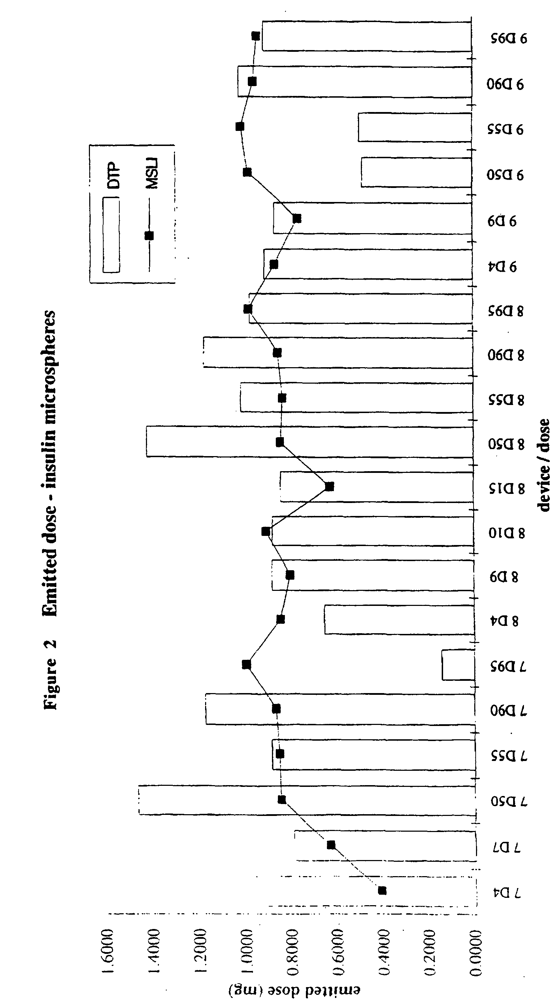

Figure 2 shows an emitted dose plot for an insulin microsphere formulation, dispensed from a Fisons Ultrahaler™, as determined using an Emitted Dose Apparatus (Multi-stage Liquid Impinger (MSLI)) in which the amount of dose to the MSLI, and the dose to patient (DTP; calculated from the MSLI), are plotted against dose number/device number.

### Example 1

### Preparation of 50:50 (w/w) insulin:starch microspheres

1 g of soluble potato starch, (Sigma, Poole, UK) was dissolved in 20 ml of distilled water. The starch was dissolved by heating the mixture to 90°C, with continuous mechanical stirring, then allowed to cool to 30°C without assistance.

1 g of zinc insulin (Novo-Nordisk, Denmark) was dissolved in 10 ml of 0.1 N HCl, with continuous mechanical stirring. The pH of the solution was then adjusted to pH 7.2 by dropwise addition of 0.1 N NaOH.

The starch solution and insulin solutions were combined and mechanically stirred for ten minutes. The pH of this solution was 7.1. The solution was spray-dried using a LabPlant SD-05 spray dryer (LabPlant, Huddersfield, UK) with the following process conditions: solution flow rate 5 ml/min, atomising nozzle diameter 0.5 mm, inlet air temperature 120°C, outlet air temperature 70°C, drying airflow 50% setting.

The collected microspheres were examined by light microscopy. The particles were spherical and had a particle size in the range 3-8 *µ*m.

### Example 2

### Preparation of 1.18% w/w calcitonin:hydroxyethyl starch (HES) microspheres

0.5 g of HES (Mw 200000; Fresenius, Austria) was dissolved in 20 ml of distilled water. 0.006 g of salmon calcitonin (sCT; Peptech) was dissolved in 10 ml of distilled water.

The HES and sCT solutions were combined and mixed. The solution was spray-dried using a LabPlant SD-05 spray drier (LabPlant, Huddersfield, UK) using the following process conditions: solution flow rate 8 ml/min, atomising nozzle diameter 0.5 mm, inlet air temperature 155°C, outlet air temperature 79-81°C, drying airflow 19 m³/h, atomising air pressure 1.9 bar.

The collected microspheres were examined by optical and scanning electron microscopy. Spherical particles with a particle size in the range 2-5 *µ*m were observed.

### Example 3

### Preparation of 20% w/w insulin:HES microspheres

16.212 g of HES (Mw 200000; Fresenius, Austria) was dissolved in 250 ml of distilled water. 3.788 g of zinc insulin (Lilly) was dissolved in 50 ml of 0.1 N HCl with continuous mechanical stirring. The pH of the solution was then adjusted to pH 7.2 by dropwise addition of 0.1 N NaOH.

The HES and insulin solutions were combined and made up to a final volume of 600 ml. The solution was spray-dried using a LabPlant SD-05 spray drier (LabPlant. Huddersfield. UK) using the following process conditions. Solution flow rate 8 ml/min. atomising nozzle diameter 0.5 mm. inlet air temperature 175°C, outlet air temperature 75-85°C, drying airflow 19 m³/h, atomising air pressure 1.9 bar.

The collected microspheres were examined by optical microscopy. Spherical particles with a particle size in the range 2-5 *µ*m were observed.

### Example 4

### Preparation of 5% w/w terbutaline:HES microspheres

A HES stock solution was prepared by dissolving 25.0 g of HES in 200 ml of ultrapure water. A terbutaline stock solution was prepared by dissolving 1250 mg of terbutaline sulphate in 200 ml of ultrapure water.

200 ml of each of the stock solutions were then made up to 500 ml with ultrapure water. The solution was spray-dried using a LabPlant SD-05 Spray Drier (LabPlant, Huddersfield, UK) using the following process conditions:

| | |
|---|---|
| Inlet air temperature | 175°C |
| Outlet air temperature | 75 - 85°C |
| Pump speed | 4 - 5 |
| Airflow | 20 units |
| Atomizing pressure | 1.9 bar |
| Nozzle size | 0.5 mm |

The microspheres were collected and had a spherical appearance. The yield was 40%.

### Example 5

### Preparation of 50% w/w morphine:HES microspheres

A HES stock solution was prepared by dissolving 2.0 g of HES in 200 ml of ultrapure water. A morphine stock solution was prepared by dissolving 2667 mg of morphine sulphate (equivalent to 2000 mg morphine base) in 200 ml of ultrapure water.

200 ml of each of the stock solutions were then made up to 500 ml with ultrapure water. The solution was spray-dried using a LabPlant SD-05 Spray Drier (LabPlant, Huddersfield, UK) using the following process conditions:

| | |
|---|---|
| Inlet air temperature | 175°C |
| Outlet air temperature | 75 - 85°C |
| Pump speed | 4 - 5 |
| Airflow | 20 units |
| Atomizing pressure | 1.9 bar |
| Nozzle size | 0.5 mm |

The microspheres were collected and had a spherical appearance. The yield was 18%.

### Example 6

### Preparation of 10% w/w human growth hormone:carboxymethyl cellulose microspheres

A carboxymethyl cellulose stock solution was prepared by dissolving 900 mg of carboxymethyl cellulose in 25 ml of ultrapure water. A human growth hormone (hGH) stock solution was prepared by dissolving 100 mg of hGH in 25 ml of ultrapure water.

25 ml of each of the stock solutions were then made up to 100 ml with ultrapure water. The solution was spray-dried using a LabPlant SD-05 Spray Drier (LabPlant, Huddersfield, UK) using the following process conditions:

| | |
|---|---|
| Inlet air temperature | 175°C |
| Outlet air temperature | 75 - 85 °C |
| Pump speed | 4 - 5 |
| Airflow | 20 units |
| Atomizing pressure | 1.9 bar |
| Nozzle size | 0.5 mm |

The microspheres were collected and had a spherical appearance. The yield was 42%.

### Example 7

### In vitro characterisation of insulin:HES microspheres delivered from a Dry Powder Device

The aerodynamic properties of the microspheres from Example 3 above were characterised *in vitro* using an Astra-type four stage liquid impinger (Copley Instruments, Nottingham, UK). The instrument was operated at a flowrate of 60 l/min using water as the collection fluid. 150 mg of insulin:HES microspheres were loaded into the reservoir of a powder aerosol device (Valois Prohaler). The firing chamber was primed and ten shots were fired into the impinger. Each shot delivered approximately 5 mg of microspheres. The distribution of the collected material in the impinger is shown in Table 1.

**Table 1.**

| **Distribution of insulin microsphere formulation in an impinger, fired from a dry powder device.** | | |
|---|---|---|
| Size of cut off (micron) | % Insulin | |
| | Run 1 | Run 2 |
| Throat | 11.5 | 21.6 |
| > 6.8 | 19.0 | 20.2 |
| < 6.8 | 69.5 | 58.2 |

The high percentage of respirable particles below 6.8 microns is notable.

### Example 8

### In vitro characterisation of 5% w/w terbutaline:HES microspheres delivered from a Dry Powder Device

The microspheres from Example 4 above were loaded into preweighed dosing chambers of a Valois Prohaler System and the aerodynamic properties of the microspheres were characterised *in vitro* using an Astra-Draco Multistage Impinger (Copley Instruments, Nottingham, UK). The instrument was operated at a flowrate of 60 l/min using water as the collection fluid. The firing chamber was primed and ten shots were fired into the impinger. Each shot delivered approximately 2.5 mg of microspheres. The drug content at each stage was measured by HPLC. The distribution of the collected material in the impinger is shown in Table 2.

**Table 2**

| **Distribution of terbutaline microsphere formulation in an impinger, fired from a dry powder device.** | |
|---|---|
| Size of cut off (micron) | % terbutaline sulphate |
| Throat | 29.6 |
| > 6.8 | 16.6 |
| < 6.8 | 54.1 |

The high percentage of respirable particles below 6.8 microns is notable.

### Example 9

### In vitro characterisation of 50% w/w morphine:HES microspheres delivered from a Dry Powder Device

The microspheres from Example 5 above were loaded into preweighed dosing chambers of a Valois Prohaler System and the aerodynamic properties of the microspheres were characterised *in vitro* using an Astra-Draco Multistage Impinger (Copley instruments, Nottingham, UK). The instrument was operated at a flowrate of 60 l/min using water as the collection fluid. The firing chamber was primed and ten shots were fired into the impinger. Each shot delivered approximately 1.6 mg of microspheres. The drug content at each stage was measured by HPLC. The distribution of the collected material in the impinger is shown in Table 3.

**Table 3**

| **Distribution of morphine microsphere formulation in an impinger, fired from a dry powder device.** | |
|---|---|
| Size of cut off (micron) | % morphine sulphate |
| Throat | 33.7 |
| > 6.8 | 21.3 |
| < 6.8 | 45.1 |

The high percentage of respirable particles below 6.8 microns is notable.

### Example 10

### In vivo characterisation of microspheres delivered from a Dry Powder Device

The insulin loaded particles prepared in Example 3 and characterised in Example 7 were evaluated in man. Doses of the insulin:HES microspheres were administered to eight healthy volunteers using a dry powder device. Both formulations were radiolabelled by surface adsorption of technetium-99m onto the insulin microspheres. The correspondence between radiolabel and insulin distribution was confirmed *in vitro* using the impinger device described in Example 7. The distribution of the formulations *in vivo* was determined by gamma scintigraphy. The results are shown in Table 4.

**Table 4**

| **Deposition of formulation *in vivo*** | |
|---|---|
| Region | % of total activity in body ± standard deviation |
| Mouth and stomach | 48.4 ± 8.0 |
| Trachea | 7.9 ± 2.7 |
| Central Lung | 21.1 ± 7.0 |
| Peripheral Lung | 19.6 ± 5.0 |
| Mean ± standard deviation (n=8). The percentage of the dose reaching the lung was 43.7%. | |

The absorption of insulin was assessed by measuring the change in plasma glucose concentration with time following dosing using a standard method. The results are shown in Table 5.

**Table 5**

| **Absorption of insulin in man (n=8) as indicated by the reduction in plasma glucose level** | |
|---|---|
| Time (mins) | Mean plasma glucose (% basal level) |
| 5 | 83.7 |
| 10 | 85.5 |
| 20 | 80.6 |
| 30 | 77.8 |
| 45 | 71.5 |
| 60 | 77.5 |
| 90 | 87.6 |
| 120 | 84.5 |
| 150 | 87.6 |
| 180 | 86.3 |
| 240 | 91.1 |

The fact that a rapid reduction in plasma glucose to a value of 72% of the basal level is obtained in 45 minutes is notable.

### Example 11

### In vitro comparison of micronised insulin and insulin microspheres delivered from a dry powder device

The aerodynamic properties of the microspheres from Example 3 above were characterised *in vitro* as before using an Astra-type four stage liquid impinger (Copley Instruments, Nottingham. UK). The impinger was operated at a flow rate of 60 l/min using water as the collection fluid.

5 mg of insulin:HES microspheres were loaded into the firing chamber of a dry powder device. The dose was then fired into the impinger. This was repeated to give a total of ten doses in insulin:HES microspheres.

For comparison, a blend of micronised insulin (size range 2-5 *µ*m) (Lilly, Indianapolis, USA) and anhydrous lactose (45-150 *µ*m) was prepared. This contained 10% w/w insulin. 10 mg aliquots of this blend were loaded into the firing chamber of the same dry powder device and the test conducted as described above.

The distribution of the collected material in the impinger is shown in Table 6.

**Table 6**

| **Distribution of insulin:HES microspheres and insulin:lactose powder blend formulations in the 4 stage impinger when fired from a dry powder device** | | | | |
|---|---|---|---|---|
| Size of cut off (micron) | Insulin micronised | | Insulin in microspheres | |
| | Run 1 | Run 2 | Run 1 | Run 2 |
| Throat | 43.2 | 12.7 | 14.4 | 12.7 |
| > 6.8 | 29.2 | 47.4 | 15.8 | 16.8 |
| < 6.8 | 27.6 | 39.9 | 69.8 | 70.6 |

The fine particle fraction, defined as the amount greater than 6.8 microns, is substantially higher for the microsphere formulation as compared to the simple blend of micronised insulin and lactose.

### Example 12

### In vivo comparison of micronised insulin and insulin microspheres

Doses of both the insulin:HES microspheres and the powder blend as characterised in Example 11 were administered to 4 healthy volunteers using a dry powder device.

The absorption of insulin was assessed by measuring the change in plasma glucose concentration with time following dosing using standard methods to measure plasma glucose levels. The results are shown in Table 7.

**Table 7**

| **Absorption of insulin in man (n=4) as indicated by the reduction in plasma glucose level** | | |
|---|---|---|
| Time (mins) | Microsphere formulation | Micronised insulin formulation |
| 5 | 92.0 | 91.5 |
| 10 | 90.3 | 87.3 |
| 20 | 89.3 | 98.0 |
| 30 | 87.3 | 99.8 |
| 45 | 80.0 | 93.5 |
| 60 | 80.8 | 89.3 |
| 90 | 86.2 | 80.3 |
| 120 | 91.5 | 84.5 |
| 150 | 93.5 | 87.5 |
| 180 | 94.5 | 90.0 |
| 240 | 98.9 | 93.0 |

It will be seen that, as in Example 10. the insulin when delivered in the starch microsphere system provides an earlier reduction in plasma glucose (time - minimum 45 mins) than the single formulation where the micronised insulin is mixed with lactose before administration (time - minimum 90 mins).

### Example 13

### In vitro comparison of micronised insulin and insulin microspheres delivered from a Fisons Ultrahaler™ dry powder device

The Ultrahaler was loaded with either insulin microspheres mixed with lactose or micronized insulin mixed with lactose. The two blends were prepared in the following way: 12.96 g of 50:50 insulin microspheres, prepared as in Example 3, or 6.56 g of micronized insulin (Lilly, Indianapolis, USA) were mixed with 54.0 g of anhydrous lactose in a Turbula T2C mixer set at speed 2 for 5 minutes, after which the powders were sieved through a 355 *µ*m sieve before being remixed for a further 5 minutes in the Turbula mixer.

The two blends were packed into Ultrahaler™ devices and the formulations were evaluated for emitted weight uniformity, emitted dose, and distribution, in an Astra Draco Multistage Liquid Impinger.

*Emitted Weight Uniformity:* The evaluation was carried out on three devices using an Emitted Weight Apparatus. A Labweigh computer programme was used to electronically capture the data. The flow rate was set at 60 l/min. For every dose the Ultrahaler™ was held in the Apparatus for 4 seconds. The filter paper was changed every five doses. The emitted mean weights for the two formulations were 18.75 mg and 14.45 mg for the micronized insulin and the insulin microsphere formulations respectively, with comparable standard deviations. This is consistent with the powder densities.

*Emitted Dose:* The evaluation was carried out on three devices using an Emitted Dose Apparatus. Single shot emitted dose studies were carried out. The doses were individually dispensed into the emitted dose collection apparatus. The flow rate was set at 60 l/min. The device was held in the apparatus for 4 seconds to allow 4 litres of air to flow through the device.

Each tube was washed several times with the same 20 ml of water. A sample was analysed for insulin content by HPLC. The results are shown in Figure 1 and Figure 2. It can be seen that the microsphere formulation clearly improved the uniformity of the emitted dose as compared to the micronized insulin formulation.

*Multistage Impinger Studies:* The aerodynamic properties of the micronized insulin and insulin microsphere formulations were characterised and compared *in vitro* by firing into an Astra-Draco Multistage Liquid Impinger (Copley Instruments, Nottingham, UK). The instrument was operated at a flow rate 60 l/min using water as the collection fluid. The firing chamber of the powder device was primed and two shots fired into the impinger. The drug content at each stage was determined by HPLC. The results are shown in Table 8.

**Table 8**

| **Distribution of micronized insulin:lactose and insulin microsphere:lactose formulations in the four stage impinger when fired from the Ultrahaler™ dry powder device.** | | | | | | |
|---|---|---|---|---|---|---|
| Size of cut off (micron) | Micronised insulin formulation (% insulin) | | | Insulin microsphere formulation (% insulin) | | |
| | Device 1 | Device 2 | Device 3 | Device 1 | Device 2 | Device 3 |
| Throat | 37.7 | 24.7 | 19.2 | 24.3 | 18.7 | 18.9 |
| > 6.8 | 32.3 | 41.4 | 44.0 | 30.3 | 38.8 | 31.9 |
| < 6.8 | 30.1 | 33.9 | 36.8 | 45.4 | 42.5 | 49.2 |

The improved percentage of respirable particles below 6.8 *µ*m for the microsphere formulation as compared to the micronized insulin formulation is notable.

## Claims

1. A composition for the delivery of pharmacological agents to the respiratory tract of a mammal to provide peripheral deposition and systemic uptake wherein the therapeutic agent is incorporated into a polysaccharide through a process of spray drying a mixture of said agent and polysaccharide, which polysaccharide is either in aqueous solution or in the aqueous phase of an emulsion, and wherein the polysaccharide is selected from soluble starch, amylodextrin, hydroxyethyl starch, polyglucosamine, amylopectin, amylose, dextran, pullulan, carboxymethyl cellulose, carboxymethyl pullulan, diethylaminoethyldextran or a mucopolysaccharide.

2. A composition as described in Claim 1 wherein the pharmacological agent is a polypeptide or protein intended for local or systemic treatment.

3. A composition as described in Claim 1 wherein the pharmacological agent is insulin, calcitonin, parathyroid hormone, a leutinising hormone releasing hormone, or analogue thereof, an interferon, desmopressin, superoxide dismutase, leptin, erythropoietin, somatostatin, colony stimulating factor (G-CSF, GM-CSF), cholecystokinin or growth hormone.

4. A composition as described in Claim 3 wherein the pharmacological agent is insulin or calcitonin.

5. A composition as described in Claim 4 wherein the pharmacological agent is insulin.

6. A composition as described in Claim 1 wherein the pharmacological agent is a low molecular weight heparin.

7. A composition as described in Claim 1 wherein the pharmacological agent is an oligonucleotide or DNA.

8. A composition as described in Claim 1 wherein the pharmacological agent is a polar analgesic agent, or a polar metabolite thereof.

9. A composition as described in Claim 8 wherein the polar analgesic agent is morphine.

10. A composition as described in Claim 8 wherein the polar metabolite is morphine-6-glucuronide.

11. A composition as described in any one of Claims 1 to 10 wherein the particles are between 0.1 and 10 microns in size.

12. A composition as described in any one of Claims 1 to 11 wherein the particles provide an immediate release of the pharmacological agent once deposited in the lungs.

13. A composition as described in any one of Claims 1 to 11 wherein the addition of a crosslinking agent or other excipients provides a controlled release of the pharmacological agent once deposited in the lungs.

14. The use of a composition according to any one of Claims 1 to 13 in the manufacture of a medicament for use in the improved systemic delivery of pharmacological agents to a mammal by the respiratory tract.

15. A composition according to any one of Claims 1 to 13 for use in the improved systemic delivery of pharmacological agents to a mammal by the respiratory tract.

16. A method for preparing microspheres for the delivery of pharmacological agents to the respiratory tract of a mammal wherein the said agent is incorporated into a microsphere using a one step process where the drug is mixed in solution with a soluble polysaccharide and thereafter particles formed through a process of spray drying, **characterised in that** the polysaccharide is selected from soluble starch, amylodextrin, hydroxyethyl starch, polyglucosamine, amylopectin, amylose, dextran, pullulan, carboxymethyl cellulose, carboxymethyl pullulan, diethylaminoethyldextran or a mucopolysaccharide.

17. A microsphere obtainable by the method of Claim 16.

## Patentansprüche

1. Substanz zur Abgabe von pharmakologischen Wirkstoffen an die Atemwege eines Säugetiers oder Menschen zur Erzielung einer peripheren Ablagerung und einer systemischen Aufnahme, wobei der therapeutische Wirkstoff in ein Polysaccharid durch ein Verfahren aufgenommen ist, bei dem eine Mischung dieses Wirkstoffes und des Polysaccharids sprühgetrocknet wird, wobei sich das Polysaccharid entweder in einer wässrigen Lösung oder in der wässrigen Phase einer Emulsion befindet, und wobei das Polysaccharid aus der Gruppe ausgewählt ist, die lösliche Stärke, Amylodextrin, Hydroxyethyl-Stärke, Polyglucosamin, Amylopectin, Amylose, Dextran, Pullulan, Carboxymethylzellulose, Carboxymethylpullulan, Diethylaminoethyldextran oder ein Mucopolysaccharid umfaßt.

2. Substanz nach Anspruch 1, bei der der pharmakologische Wirkstoff ein Polypeptid oder Protein ist, das für eine örtliche oder systemische Behandlung vorgesehen ist.

3. Substanz nach Anspruch 1, bei der der pharmakologische Wirkstoff Insulin, Calcitonin, ein Parathyroid-Hormon, ein ein Leutinisierungshormon freisetzendes Hormon oder eine analoge Substanz hierzu, ein Interferon, ein Desmopressin, Superoxyd-Dismutase, ein Leptin, Erythropoietin, Somatostatin, ein kolonie-stimulierender Faktor (G-CSF, GM-CSF), Cholecystokinin oder ein Wachstumshormon ist.

4. Substanz nach Anspruch 3, bei der der pharmakologische Wirkstoff Insulin oder Calcitonin ist.

5. Substanz nach Anspruch 4, bei der der pharmakologische Wirkstoff Insulin ist.

6. Substanz nach Anspruch 1, bei der der pharmakotogische Wirkstoff ein Heparin mit niederem Molekulargewicht ist.

7. Substanz nach Anspruch 1, bei der der pharmakologische Wirkstoff ein Oligonucleotid oder DNA ist.

8. Substanz nach Anspruch 1, bei der der pharmakologische Wirkstoff ein polarer schmerzstillender Wirkstoff oder ein polarer Methabolit hiervon ist.

9. Substanz nach Anspruch 8, bei der der polare schmerzstillende Wirkstoff Morphin ist.

10. Substanz nach Anspruch 8, bei der der polare Methabolit Morphin-6-Glucuronid ist.

11. Substanz nach einem der Ansprüche 1 bis 10, bei der die Teilchen eine Größe zwischen 0,1 und 10 µm besitzen.

12. Substanz nach einem der Ansprüche 1 bis 11, bei der die Teilchen eine sofortige Freisetzung des pharmakologischen Wirkstoffes zeigen, sobald sie in der Lunge abgeschieden worden sind.

13. Substanz nach einem der Ansprüche 1 bis 11, bei der das Hinzufügen eines Quervernetzungswirkstoffes oder eines anderen Arzneimittelträgers für eine kontrollierte Freisetzung des pharmakologischen Wirkstoffes sorgt, sobald die Substanz in der Lunge abgeschieden worden ist.

14. Verwendung einer Substanz nach einem der Ansprüche 1 bis 13 bei der Herstellung eines Medikamentes zum Gebrauch bei der verbesserten systemischen Abgabe von pharmakologischen Wirkstoffen an ein Säugetier oder einen Menschen über die Atmungsorgane.

15. Substanz nach einem der Ansprüche 1 bis 13 zur Verwendung bei der verbesserten systemischen Abgabe eines pharmakologischen Wirkstoffes an ein Säugetier oder einen Menschen über die Atmungsorgane.

16. Verfahren zur Herstellung von Mikrokügelchen zur Abgabe eines pharmakologischen Wirkstoffes an die Atmungsorgane eines Säugetiers oder Menschen, bei dem der Wirkstoff in ein Mikrokügelchen unter Verwendung eines einstufigen Verfahrens aufgenommen wird, bei dem das Medikament in Lösung mit einem löslichen Polysaccharid gemischt und danach Teilchen durch ein Sprühtrocknungsverfahren gebildet werden, **dadurch gekennzeichnet, daß** das Polysaccharid aus der Gruppe ausgewählt ist, die lösliche Stärke, Amylodextrin, Hydroxyethyl-Stärke, Polyglucosamin, Amylopectin, Amylose, Dextran, Pullulan, Carboxymethyl-Zellulose, Carboxymethyl-Pullulan, Diethylaminoethyldextran oder ein Mucopolysaccharid umfaßt.

17. Mikrokügelchen, welches nach dem Verfahren aus Anspruch 16 erhalten wird.

## Revendications

1. Composition pour l'administration d'agents pharmacologiques dans les voies respiratoires d'un mammifère pour permettre le dépôt périphérique et la captation systémique, dans laquelle l'agent thérapeutique est incorporé dans un polysaccharide par un procédé de séchage par pulvérisation d'un mélange dudit agent et dudit polysaccharide, le polysaccharide étant soit en solution aqueuse soit dans la phase aqueuse d'une émulsion et dans laquelle le polysaccharide est choisi parmi l'amidon soluble, l'amylodextrine, l'hydroxyéthyl-amidon, la polyglucosamine, l'amylopectine, l'amylose, la dextrane, le pullulane, la carboxyméthylcellulose; le carboxyméthylpullulane, la diéthylaminoéthyldextrane ou un mucopolysaccharide.

2. Composition selon la revendication 1 dans laquelle l'agent pharmacologique est un polypeptide ou une protéine destinée au traitement local ou systémique.

3. Composition selon la revendication 1 dans laquelle l'agent pharmacologique est de l'insuline, de la calcitonine, de l'hormone parathyroidienne, une hormone de libération de la lutéinostimuline ou un analogue de cette hormone, un interféron, de la desmopressine, de la superoxide-dismutase, de la leptine, de l'érythropoiétine, de la somatostatine, un facteur stimulant la formation de colonies (G-CSF, GM-CSF), de la cholécystokinine ou de l'hormone de croissance.

4. Composition selon la revendication 3 dans laquelle l'agent pharmacologique est de l'insuline ou de la calcitonine.

5. Composition selon la revendication 4 dans laquelle l'agent pharmacologique est de l'insuline.

6. Composition selon la revendication 1 dans laquelle l'agent pharmacologique est une héparine de bas poids moléculaire.

7. Composition selon la revendication 1 dans laquelle l'agent pharmacologique est un oligonucléotide ou de l'ADN.

8. Composition selon la revendication 1 dans laquelle l'agent pharmacologique est un agent analgésique polaire ou un métabolite polaire de celui-ci.

9. Composition selon la revendication 8 dans laquelle l'agent analgésique polaire est de la morphine.

10. Composition selon la revendication 8 dans laquelle le métabolite polaire est de la morphine-6-glucuronide.

11. Composition selon l'une quelconque des revendications 1 à 10 dans laquelle les particules ont une taille comprise entre 0,1 et 10 microns.

12. Composition selon l'une quelconque des revendications 1 à 11 dans laquelle les particules permettent une libération immédiate de l'agent pharmacologique une fois déposées dans les poumons.

13. Composition selon l'une quelconque des revendications 1 à 11 dans laquelle l'ajout d'un agent réticulant ou d'autres excipients permet une libération contrôlée de l'agent pharmacologique une fois déposé dans les poumons.

14. Utilisation d'une composition selon l'une quelconque des revendications 1 à 13 dans la fabrication d'un médicament pour l'utilisation dans le cadre de l'administration systémique améliorée d'agents pharmacologiques à un mammifère par les voies respiratoires.

15. Composition selon l'une quelconque des revendications 1 à 13 pour l'utilisation dans le cadre de l'administration systémique améliorée d'agents pharmacologiques à un mammifère par les voies respiratoires.

16. Méthode de préparation de microsphères pour l'administration d'agents pharmacologiques dans les voies respiratoires d'un mammifère dans laquelle ledit agent est incorporé dans une microsphère par un procédé en une étape au cours duquel le médicament est mélangé en solution avec un polysaccharide soluble puis les particules formées par un procédé de séchage par pulvérisation **caractérisée en ce que** le polysaccharide est choisi parmi l'amidon soluble, l'amylodextrine, l'hydroxyéthyl-amidon, la polyglucosamine, l'amylopectine, l'amylose, la dextrane, le pullulane, la carboxyméthylcellulose, le carboxyméthylpullulane, la diéthylaminoéthyldextrane ou un mucopolysaccharide.

17. Microsphëre pouvant être obtenue par la méthode selon la revendication 16.
